(19) Europäisches Patentamt

European Patent Office

Office européen des brevets

(11) **EP 4 238 603 A1**

(12) **EUROPEAN PATENT APPLICATION**
published in accordance with Art. 153(4) EPC

(43) Date of publication:
06.09.2023 Bulletin 2023/36

(21) Application number: 22854498.7

(22) Date of filing: 23.08.2022

(51) International Patent Classification (IPC):
*A61M 21/00* (2006.01)      *A61M 21/02* (2006.01)
*A61B 5/0205* (2006.01)      *A61B 5/05* (2021.01)
*G06F 17/40* (2006.01)      *G06F 17/00* (2019.01)
*G06F 16/60* (2019.01)

(52) Cooperative Patent Classification (CPC):
(C-Sets available)
**A61M 21/02;** A61M 2021/0027; A61M 2205/332;
A61M 2205/505; A61M 2230/06; A61M 2230/63

(Cont.)

(86) International application number:
**PCT/CN2022/114355**

(87) International publication number:
**WO 2023/130737 (13.07.2023 Gazette 2023/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 06.01.2022   CN 202210008594

(71) Applicant: **Anhui Huami Health Technology Co.,
Ltd.**
**Hefei, Anhui 243000 (CN)**

(72) Inventors:
• **HUANG, Wang**
**Hefei, Anhui 243000 (CN)**

• **WANG, Kongqiao**
**Hefei, Anhui 243000 (CN)**
• **YAN, Jinian**
**Hefei, Anhui 243000 (CN)**
• **ZHU, Guokang**
**Hefei, Anhui 243000 (CN)**
• **MENG, Zi**
**Hefei, Anhui 243000 (CN)**
• **ZHANG, Cong**
**Hefei, Anhui 243000 (CN)**
• **YU, Yi**
**Hefei, Anhui 243000 (CN)**

(74) Representative: **Angerhausen, Christoph
Boehmert & Boehmert
Anwaltspartnerschaft mbB
Pettenkoferstrasse 22
80336 München (DE)**

(54) **METHOD AND APPARATUS FOR AUDIO PLAYING**

(57)    The present disclosure provides a method, an apparatus, a wearable device and a storage medium for audio playback, and relates to the fields of machine learning, deep learning, intelligent wearable, etc. The method includes: measured vital sign data of a user within a first time range is obtained; a vital sign prediction is performed based on the measured vital sign data within the first time range, to obtain predicted vital sign data of the user within a second time range after the first time range; one or more target audio parameters are determined based on the predicted vital sign data and user attribute information of the user; and matched target audio data is obtained based on the one or more target audio parameters.

**(Cont. next page)**

EP 4 238 603 A1

obtaining measured vital sign data of a user within a first time range — S101

performing a vital sign prediction based on the measured vital sign data within the first time range, to obtain predicted vital sign data of the user within a second time range after the first time range — S102

determining one or more target audio parameters based on the predicted vital sign data and user attribute information of the user — S103

obtaining matched target audio data based on the one or more target audio parameters — S104

FIG. 1

(52) Cooperative Patent Classification (CPC): (Cont.)

C-Sets
A61M 2230/06, A61M 2230/005;
A61M 2230/63, A61M 2230/005

**Description**

**CROSS-REFERENCE TO RELATED APPLICATION**

The disclosure claims the priority to Chinese Patent Application No. 202210008594.5 filed to China National Intellectual Property Administration on January 06, 2022, the entire content of which is incorporated herein by reference.

**TECHNICAL FIELD**

The present disclosure relates to the field of computer technologies, in particular to the field of machine learning, deep learning, smart wearables, etc., in particular to a method and an apparatus for audio playback, a wearable device and a storage medium.

**BACKGROUND**

With the development and change of modern society, the pace of work and life is accelerating, which also puts higher requirements for people's spiritual and physical adaptability. More and more people are faced with sub-health problems such as insomnia, obesity, depression, and so on, which gives birth to a variety of ways to regulate and improve sleep and/or health. Audio playback is a method to adjust and improve users' health based on vital sign data.

However, in actual playback, audio often does not match the users' signs, resulting in poor improvement of the users' health.

**SUMMARY**

The disclosure is intended to solve one of technical problems in the related art at least to a certain extent.

In order to achieve the above purpose, the first aspect of the present disclosure provides a method for audio playback, including:

obtaining measured vital sign data of a user within a first time range;
performing a vital sign prediction based on the measured vital sign data within the first time range, to obtain predicted vital sign data of the user within a second time range after the first time range;
determining one or more target audio parameters based on the predicted vital sign data and user attribute information of the user; and
obtaining matched target audio data based on the one or more target audio parameters.

In some implementations, obtaining measured vital sign data of the user within the first time range includes: obtaining the measured vital sign data of the user within the first time range based on user scenario information, in which the user scenario information is used for indicating health requirements of the user.

In some implementations, obtaining measured vital sign data of the user within the first time range based on the user scenario information includes:

in response to the user scenario information indicating a sleep aid scenario or a focus scenario, obtaining heart rate data and activity data of the user within the first time range; or
in response to the user scenario information indicating a weight loss scenario, obtaining at least one of activity data, motion data, heart rate data, or positioning data of the user within the first time range; or
in response to the user scenario information indicating an emotion regulation scenario, obtaining heart rate variability of the user within the first time range.

In some implementations, performing the vital sign prediction based on the measured vital sign data within the first time range, to obtain the predicted vital sign data of the user within the second time range after the first time range includes:

performing feature extraction processing on the measured vital sign data within the first time range, to obtain first feature data;
performing fusing processing on the first feature data and the user attribute information, to obtain second feature data; and
performing the vital sign prediction based on the second feature data, to obtain the predicted vital sign data within the second time range.

In some implementations, determining one or more target audio parameters based on the predicted vital sign data and user attribute information of the user includes:

> determining a target physiological parameter of the user; and
> determining the one or more target audio parameters based on the predicted vital sign data, the target physiological parameter and user attribute information.

In some implementations, determining the target physiological parameter of the user includes:
determining the target physiological parameter of the user based on at least one of user scenario information or historical vital sign data of the user.

In some implementations, determining the target physiological parameter of the user includes:

> in response to the user scenario information indicating a sleep aid scenario or a focus scenario, determining target activeness of the user; or
> in response to the user scenario information indicating a weight loss scenario, determining at least one of target activeness, target activity amount and target heart rate of the user; or
> in response to the user scenario information indicating an emotion regulation scenario, determining heart rate variability of the user.

In some implementations, the target physiological parameter includes activeness;

> determining the target physiological parameter of the user includes:
> obtaining historical heart rate data and historical activity data of the user within a third time range corresponding to the first time range;
> determining the historical activeness of the user based on the historical heart rate data and the historical activity data of the user; and
> determining target activeness of the user based on the historical activeness of the user, the target activeness is less than the historical activeness.

In some implementations, determining one or more target audio parameters based on the predicted vital sign data and user attribute information of the user includes:
inputting the predicted vital sign data, the target physiological parameter and the user attribute information into a regression model, to obtain the one or more target audio parameters outputted by the regression model, the one or more target audio parameters include target audio loudness and target rhythm.

In some implementations, the one or more target audio parameters include a target audio modulation parameter;

> obtaining matched target audio data based on the one or more target audio parameters includes:

>> sending the target audio modulation parameter to a server, so as to enable the server to query target audio data matching the target audio modulation parameter; and
>> obtaining the target audio data sent by the server.

According to the method for audio playback in the implementation of the present disclosure, measured vital sign data of a user within a first time range is obtained; a vital sign prediction is performed based on the measured sign data within the first time range, to obtain predicted sign data of the user within a second time range after the first time range; one or more target audio parameters are determined based on the predicted vital sign data and user attribute information of the user; and matched target audio data is obtained based on the one or more target audio parameters. Since the one or more target audio parameters are determined based on the predicted vital sign data, the target physiological parameter and the user attribute information, the target audio data matched with the one or more target audio parameters can be effective for adjusting sleep or health for the user, so as to solve the technical problem of the related technologies that actual audio playback does not match requirements of the user and the adjustment effect is poor.

In order to achieve the above purpose, a second aspect of the present disclosure provides a method for audio playback includes:

> determining a target physiological parameter of a user based on user scenario information, in which the user scenario information is used for indicating health requirements of the user;
> determining one or more target audio parameters based on the target physiological parameter of the user; and
> obtaining matched target audio data based on the one or more target audio parameters.

In some implementations, determining a target physiological parameter of the user based on user scenario information includes:

in response to the user scenario information indicating a sleep aid scenario or a focus scenario, determining target activeness of the user; or
in response to the user scenario information indicating a weight loss scenario, determining at least one of target activeness, target activity amount and target heart rate of the user; or
in response to the user scenario information indicating an emotion regulation scenario, determining heart rate variability of the user.

In some implementations, determining one or more target audio parameters based on the target physiological parameter of the user includes:

obtaining measured vital sign data of the user within a first time range based on the user scenario information;
performing a vital sign prediction based on the measured vital sign data within the first time range, to obtain predicted vital sign data of the user within a second time range after the first time range; and
determining the one or more target audio parameters based on the predicted vital sign data, the target physiological parameter and user attribute information.

In some implementations, obtaining measured vital sign data of the user within a first time range based on the user scenario information includes:

in response to the user scenario information indicating a sleep aid scenario or a focus scenario, obtaining heart rate data and activity data of the user within the first time range; or
in response to the user scenario information indicating a weight loss scenario, obtaining at least one of activity data, motion data, heart rate data, or positioning data of the user within the first time range; or
in response to the user scenario information indicating an emotion regulation scenario, obtaining heart rate variability of the user within the first time range.

In some implementations, performing a vital sign prediction based on the measured vital sign data within the first time range, to obtain predicted vital sign data of the user within a second time range after the first time range includes:

performing feature extraction processing on the measured vital sign data within the first time range, to obtain first feature data;
performing fusing processing on the first feature data and the user attribute information, to obtain second feature data; and
performing the vital sign prediction based on the second feature data, to obtain the predicted vital sign data within the second time range.

In some implementations, determining the one or more target audio parameters based on the predicted vital sign data, the target physiological parameter and the user attribute information includes:

inputting the predicted vital sign data, the target physiological parameter and the user attribute information into a regression model, to obtain the one or more target audio parameters outputted by the regression model.
In some implementations, the one or more target audio parameters include a target audio modulation parameter; obtaining matched target audio data based on the one or more target audio parameters includes:

sending the target audio modulation parameter to a server, so as to enable the server to query target audio data matching the target audio modulation parameter; and
obtaining the target audio data sent by the server.

According to the method for audio playback in the implementation of the present disclosure, the target physiological parameter is determined based on user scenario information; one or more target audio parameters are determined based on the target physiological parameter of the user; and matched target audio data is determined based on the one or more target audio parameters. The user scenario information is used for indicating health requirements of the user. In this way, the one or more target audio parameters are determined which help the user to achieve the target physiological parameter by a matching model of target physiological parameter and one or more target audio parameters based on the health requirements of the user. According to this active modulation method, a target audio is effectively determined based on health requirements of the user, so as to better improve health status of the user.

In order to achieve the above purpose, an apparatus for audio playback is provided in a third aspect of the present disclosure, which includes:

a collecting module, configured to obtain measured vital sign data of a user within a first time range;
a predicting module, configured to perform a vital sign prediction based on the measured vital sign data within the first time range, to obtain predicted vital sign data of the user within a second time range after the first time range;
a first determining module, configured to determine one or more target audio parameters based on the predicted vital sign data and user attribute information of the user; and
an acquiring module, configured to obtain matched target audio data based on the one or more target audio parameters.

In order to achieve the above purpose, a wearable device is provided in a fourth aspect of the disclosure, and includes: at least one processor; and a memory communicatively coupled to the at least one processor; the memory is stored with instructions executable by the at least one processor, the instructions are performed by the at least one processor, the at least one processor is caused to perform the method for audio playback as described in the first aspect of the disclosure or the method for audio playback as described in the second aspect of the disclosure.

In order to achieve the above purpose, a computer readable storage medium stored with computer instructions is provided in a fifth aspect of the disclosure. The computer instructions are configured to execute the method for audio playback as described in the first aspect and the method for audio playback as described in the second aspect by the computer.

In order to achieve the above purpose, a computer program product is provided in a sixth aspect of the disclosure, and includes a computer program, the computer program executes the method for audio playback as described in the first aspect and the method for audio playback as described in the second aspect when executed by a processor.

Additional aspects and advantages of the present disclosure will be set forth in part in the following description, and in part will become obvious from the following description, or may be learned by practice of the disclosure.

## BRIEF DESCRIPTION OF THE DRAWINGS

FIG. 1 is a flowchart illustrating a method for audio playback provided in an implementation of the present disclosure;
FIG. 2 is a schematic diagram of a principle of audio playback provided in the implementations of the present disclosure;
FIG. 3 is a flowchart of a method for audio playback provided in another implementation of the present disclosure;
FIG. 4 is a schematic diagram of a prediction model provided in the implementation of the present disclosure;
FIG. 5 is a schematic diagram of a regression model provided in the implementation of the present disclosure;
FIG. 6 is a flowchart of a method for audio playback provided in another implementation of the present disclosure;
FIG. 7 is a structural diagram of an apparatus for audio playback provided in the implementation of the present disclosure;
FIG. 8 is a structural diagram of an apparatus for audio playback provided in another implementation of the present disclosure; and
FIG. 9 is a structural diagram of a wearable device provided in the implementation of the present disclosure.

## DETAILED DESCRIPTION

Implementations of the present disclosure are described in detail below, and examples of implementations are illustrated in the accompanying drawings, in which the same or similar labels represent the same or similar elements or elements with the same or similar functions. The implementations described below with reference to the drawings are exemplary, are intended to be configured to explain the present disclosure and are not to be construed as a limitation of the present disclosure.

At present, an audio is modulated and played based on a latest measured vital sign state of a user. However, if the audio is modulated and played based only on the latest measured vital sign state of the user, when the audio is being played, the audio is lagged behind vital sign of the user, which makes the audio do not match the vital sign of the user, and it is difficult to meet personalized modulation requirements of the user.

Therefore, in view of the above problems, the present disclosure proposes a method, an apparatus for audio playback, a wearable device and a storage medium to determine one or more target audio parameters based on predicted vital sign data, a target physiological parameter and user attribute information, which solves the problem of the prior art that the actual audio played does not match the requirements of the user and the adjustment effect is poor.

A method and an apparatus for audio playback, a wearable device and a storage medium are described referring to figures in implementations of the present disclosure.

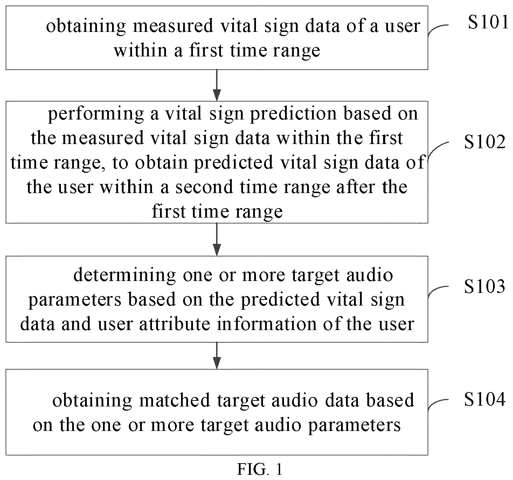

FIG. 1 is a flowchart illustrating a method for audio playback provided in one implementation of the present disclosure.

The method for audio playback is illustrated exemplarily by the apparatus for audio playback, and the apparatus for audio playback can be applied to any type of electronic devices, such as a smart phone, a tablet computer, a vehicle mounted device, a smart audio, a wearable device, etc. For ease of understanding, the method is described below with an example of being executed by a wearable device.

As illustrated in FIG. 1, the method for audio playback includes the following S101 to S104.

At S101, measured vital sign data of a user within a first time range is obtained.

The first time range is a certain moment, or a continuous period of time, or a plurality of moments distributed at a specific time interval. The first time range is a preset time range starting from a moment when the user triggers audio playback, or a preset time range before the moment when the user triggers audio playback, or a preset time range after the moment when audio is played. The duration of the first time range is set according to actual requirements.

The measured vital sign data includes one or any combination of heart rate data, activity amount data, pressure data, sleep data, emotion data and respiratory rate data.

The measured vital sign data is collected by one or more sensors set in the wearable device. A sensor set in the wearable device monitors the current vital sign status of the user in real time, and the number of the sensors set in the wearable device is one or more, such as an acceleration sensor, a heart rate sensor, etc.

In some implementations, obtaining the measured vital sign data of the user within the first time range includes: obtaining the measured vital sign data of the user within the first time range based on user scenario information.

The user scenario information indicates the health requirements of the user.

Different user scenario information corresponds to different health requirements. The health requirements of the user include one or any combination of sleep aid, focus, weight loss and emotion regulation.

In some implementations, obtaining the measured vital sign data of the user within the first time range based on user scenario information includes: in response to the user scenario information indicating a sleep aid scenario or a focus scenario, obtaining heart rate data and activity data of the user within the first time range; or, in response to the user scenario information indicating a weight loss scenario, obtaining at least one of the activity data, motion data, the heart rate data, or positioning data of the user within the first time range; or, in response to the user scenario information indicating an emotion regulation scenario, obtaining heart rate variability of the user within the first time range.

In the case that the user scenario information indicates the sleep aid scenario or the focus scenario, the measured vital sign data includes the heart rate data and/or the activity data of the user. The heart rate data and the activity data represent a relaxation state of the user. In the case that the user scenario information indicates the weight loss scenario, the measured vital sign data includes the activity data, the motion data, the heart rate data, and the positioning data. The activity data, the motion data, the heart rate data, and the positioning data represent an activity status of the user, and can represent activeness, activity amount or the like of the user. In the case that the user scenario information indicates the emotion regulation scenario, the measured vital sign data includes the heart rate variability. The heart rate variability represents emotional state of the user.

In some implementations, vital sign data of the user at a target moment is collected in the procedure.

In the implementation of the present disclosure, the vital sign data of the user includes one or more combinations of the heart rate, activity amount, pressure, sleep, emotion and respiratory rate.

In the implementation of the present disclosure, the vital sign data of the user is collected by a sensor mounted in the wearable device. The sensor mounted in the wearable device monitors the current vital sign status of the user in real time.

In some implementations, the moment when the user triggers audio playback is taken as the target moment, and the vital sign data collected at the moment when the user triggers audio playback is taken as the vital sign data of the user at the target moment.

In some implementations, the next moment after the user triggers audio playback is taken as the target moment for data collection. The number of the sensors mounted in the wearable device is one or more.

As an example, the method for audio playback is applied to a smart watch for illustrative explanation. The user is the person wearing the smart watch, and current vital sign state of the user is monitored in real time by the one or more sensors mounted in the smart watch. The target moment is the moment when the user triggers the audio playback and the vital sign data of the user at the target moment is collected.

At S 102, a vital sign prediction is performed based on the measured vital sign data within the first time range, and predicted vital sign data within a second time range after the first time range is obtained.

The duration of the second time range is the same as or different from that of the first time range.

In some implementations, the vital sign prediction is performed based on the measured vital sign data within the first time range, and the predicted vital sign data within the second time range after the first time range is obtained. Specifically, a feature extraction processing is performed on the measured vital sign data within the first time range, to obtain first feature data, the first feature data and user attribute information are fused to obtain second feature data, and the vital sign prediction is performed based on the second feature data, to obtain the predicted vital sign data within a second time range.

The user attribute information includes basic information of the user, which includes at least one of age, gender, height, weight or disease status. In some implementations, attributes of the user are related to the environment the user is in, and accordingly the user attribute information also includes environment information of the environment the user is in, where the environment information includes at least one of weather and time.

In some implementations, in the procedure, the vital sign prediction is performed based on the user vital sign data at the target moment, to obtain the predicted vital sign data at a set moment after the target moment.

In the implementations of the present disclosure, the vital sign prediction is performed on the user vital sign data at the target moment, and the predicted vital sign data at the set moment after the target moment is obtained by predicting the future natural evolution of the vital signs of the user at the target moment.

In some implementations, change patterns of the vital sign data, such as the heart rate, the activity amount and the pressure over time, are captured to perform the vital sign prediction.

At S103, one or more target audio parameters are determined based on the predicted vital sign data and the user attribute information of the user.

In some implementations, the one or more target audio parameters are determined based on the predicted vital sign data and the user attribute information of the user. Specifically, a target physiological parameter of the user is determined; and the one or more target audio parameters are determined based on the predicted vital sign data, the target physiological parameter and the user attribute information.

In some implementations, in response to the user scenario information indicating the sleep aid scenario or the focus scenario, target activeness of the user is determined. For example, if the scenario set by the user is the weight loss scenario, the corresponding target physiological parameter includes the activeness, which is used as a measure of relaxation of the user before sleep, which can help the user enter a sleep state as soon as possible.

In other implementations, in response to the user scenario information indicating the weight loss scenario, at least one of the target activeness, target activity amount or target heart rate of the user is determined. For example, in the weight loss scenario, the target activeness, the target activity amount and the target heart rate of the user are determined. The target heart rate is the heart rate that the user achieves an effect of burning fat. The target activity amount is specified by the user or determined based on historical data of the user.

In other implementations, in response to the user scenario information indicating an emotion regulation scenario, target heart rate variability of the user is determined.

The target physiological parameter includes at least one of the target activeness, the target activity amount, the target heart rate or the target heart rate variability.

If the scenario set by the user is the emotion regulation scenario, the corresponding target physiological parameter includes HRV (heart rate variability). The emotion is highly correlated with HRV A low HRV indicates a relaxed state, while a high HRV indicates a potential state of nervousness or depression. Taking HRV as the target physiological parameter can help the user adjust emotion in a timely manner. In some implementations, when it is detected that the user is in a nervous or depressed state, a lower HRV is set as the target physiological parameter.

In some implementations, the target physiological parameter of the user is determined. Specifically, the target physiological parameter of the user is determined based on at least one of the user scenario information and historical vital sign data of the user.

In some implementations, the target physiological parameter includes the activeness. The target physiological parameter of the user is determined based on at least one of the user scenario information and the historical vital sign data of the user. Specifically, historical heart rate data and historical activity data of the user within a third time range corresponding to the first time range is obtained; historical activeness of the user is determined based on the historical heart rate data and the historical activity data of the user; and the target activeness of the user is determined based on the historical activeness of the user. The target activeness is less than the historical activeness.

The historical vital sign data includes the historical heart rate data and the historical activity data. The historical vital sign data is the data of the user stored in the wearable device.

In some implementations, the one or more target audio parameters are determined based on the predicted vital sign data and the user attribute information of the user. Specifically, the predicted vital sign data, the target physiological parameter and the user attribute information are inputted into a regression model, to obtain the one or more target audio parameters outputted by the regression model. The one or more target audio parameters include target audio loudness and target rhythm.

In the implementations of the present disclosure, due to the different requirements for adjusting vital signs in different scenarios, the corresponding target physiological parameters are different in different scenarios. In some implementations, if the scenario set by the user is a weight-loss-by-exercise scenario, the corresponding target physiological parameter includes a fat burning heart rate for the user in an exercise state. The fat burning heart rate is one of metrics for measuring weight loss by exercise, which can help the user to obtain higher weight loss efficiency.

In some implementations, in the case that the scenario is set as the weight-loss-by-exercise scenario, for users not in the exercise state, rhythmic audio is played to improve their exercise interest. In some implementations, in combination with a sedentary reminder function of the wearable device, rhythmic audio is played to guide the user to do some exercises. In some implementations, audio suitable for outdoor activities is played to guide the user to perform outdoor activities.

In some implementations, there is one or more mapping relationships between the predicted vital sign data, the target physiological parameter and user attribute information with the one or more target audio parameters. In the implementations of the present disclosure, the one or more target audio parameters required are determined by querying the mapping relationship based on the predicted vital sign data, the target physiological parameter and the user attribute information determined. Thus, the one or more target audio parameters are matched with the predicted vital sign data, the target physiological parameter and the user attribute information. In some implementations, the one or more target audio parameters include the target audio loudness and the target rhythm.

At S 104, matched target audio data is obtained based on the one or more target audio parameters. In some implementations, the one or more target audio parameters include one or more target audio modulation parameters. The matched target audio data is obtained based on the one or more target audio parameters. Specifically, the one or more target audio modulation parameters are sent to a server, so as to enable the server to query the target audio data matching the one or more target audio modulation parameters, and the target audio data sent by the server is obtained.

The target audio modulation parameters include the target audio loudness and the target rhythm.

In some implementations, in this procedure, matched target audio is obtained and played according to the one or more target audio modulation parameters.

In the implementation of the present disclosure, audio elements are modulated and classified in advance, and an audio modulation library is generated and stored at the cloud side. The audio elements include a plurality of combinations of drum points, melodies, natural sounds and loudness.

In some implementations, matched audio elements are queried based on the target audio modulation parameters, namely the target audio loudness and the target rhythm, queried audio elements are synthesized to obtain the target audio, and the target audio is played.

Based on the above, the measured vital sign data of the user within the first time range is obtained. The vital sign prediction is performed based on the measured vital sign data within the first time range, to obtain the predicted vital sign data of the user within the second time range after the first time range. The one or more target audio parameters are determined based on the predicted vital sign data, the target physiological parameter and the user attribute information, and the matched target audio data is obtained based on the one or more target audio parameters. Since the one or more target audio parameters are determined based on the predicted vital sign data, the target physiological parameter and the user attribute information, the target audio data matched with the one or more target audio parameters can be effective for adjusting sleep or health for the user, thereby solving the technical problem of the related technologies that actual audio playback does not match the requirements of the user and the adjustment effect is poor.

In the other aspect of the present disclosure, a method for audio playback is provided. Specifically, the target physiological parameter of the user is determined based on the user scenario information, where the user scenario information is configured to indicate health requirements of the user, the one or more target audio parameters are determined based on the target physiological parameter of the user, and the matched target audio data is obtained based on the one or more target audio parameters.

According to the method for audio playback in the implementations of the present disclosure, the target physiological parameter is determined based on user scenario information, one or more target audio parameters are determined based on the target physiological parameter of the user, and matched target audio data is determined based on the one or more target audio parameters. The user scenario information is used for indicating the health requirements of the user. In this way, starting from the health needs of the user, the one or more target audio parameters that enable the user to achieve the target physiological parameter are quantified by a model for matching the target physiological parameter and the one or more target audio parameters. The manner of active modulation effectively determines the target audio based on the health requirements of the user, thereby improving health status of the user more effectively.

In some implementations, the target physiological parameter is determined based on the user scenario information. Specifically, in response to the user scenario information indicating the sleep aid scenario or the focus scenario, the target activeness of the user is determined. Or, in response to the user scenario information indicating the weight loss scenario, at least one of the target activeness, the target activity amount or the target heart rate of the user is determined. Or, in response to the user scenario information indicating the emotion regulation scenario, the heart rate variability of the user is determined.

In the case that the user scenario information indicates the sleep aid scenario or the focus scenario, the measured vital sign data includes the heart rate data and/or the activity data of the user. The heart rate data and the activity data represent a relaxation state of the user. In the case that the user scenario information indicates the weight loss scenario, the measured vital sign data includes the activity data, motion data, the heart rate data, and positioning data. The activity data, the motion data, the heart rate data, and the positioning data represent an activity status of the user, thereby represent activeness, the activity amount of the user or the like. In the case that the user scenario information indicates the emotion regulation scenario, the measured vital sign data includes the heart rate variability. The heart rate variability represents an emotional state of the user.

In some implementations, the one or more target audio parameters are determined based on the target physiological parameter of the user. Specifically, the measured vital sign data of the user within the first time range is determined based on the user scenario information, the vital sign prediction is performed based on the measured sign data within the first time range, to obtain predicted vital sign data of the user within the second time range after the first time range, and the one or more target audio parameters are determined based on the predicted vital sign data, the target physiological parameter and the user attribute information.

The duration of the second time range is the same as or different from that of the first time range.

In some implementations, the measured vital sign data of the user within the first time range is determined based on the user scenario information. Specifically, in response to the user scenario information indicating the sleep aid scenario or the focus scenario, the heart rate data and the activity data of the user within the first time range is obtained; or in response to the user scenario information indicating the weight loss scenario, at least one of the activity data, the motion data, the heart rate data, or the positioning data of the user within the first time range is obtained; or in response to the user scenario information indicating an emotion regulation scenario, the heart rate variability of the user within the first time range is obtained.

In some implementations, the vital sign prediction is performed based on the measured vital sign data within the first time range, the predicted vital sign data within the second time range after the first time range is obtained. Specifically, the feature extraction processing is performed on the measured vital sign data within the first time range, to obtain first feature data. The first feature data and the user attribute information are fused to obtain second feature data. The prediction is performed based on the second feature data, to obtain the predicted vital sign data within a second time range.

The user attribute information includes the basic user information, which includes at least one of the age, the gender, the height, the weight or the health status. In some implementations, the user attributes are related to the environment the user is in, so the user attribute information also includes the environment information of the environment the user is in, and the environment information includes at least one of weather or time.

In some implementations, the one or more target audio parameters are determined based on the predicted vital sign data, the target physiological parameter and user attribute information. Specifically, the predicted vital sign data, the target physiological parameter and the user attribute information are inputted into the regression model, to obtain the one or more target audio parameters outputted by the regression model.

The one or more target audio parameters include the target audio loudness and the target rhythm.

In some implementations, the one or more target audio parameters include the target audio modulation parameters.

The matched target audio data is obtained based on the one or more target audio parameters. Specifically, the target audio modulation parameters are sent to the server, so as to enable the server to query target audio data matching the target audio modulation parameter; and the target audio data sent by the server is obtained.

An example is illustrated herein to describe the above implementations more clearly.

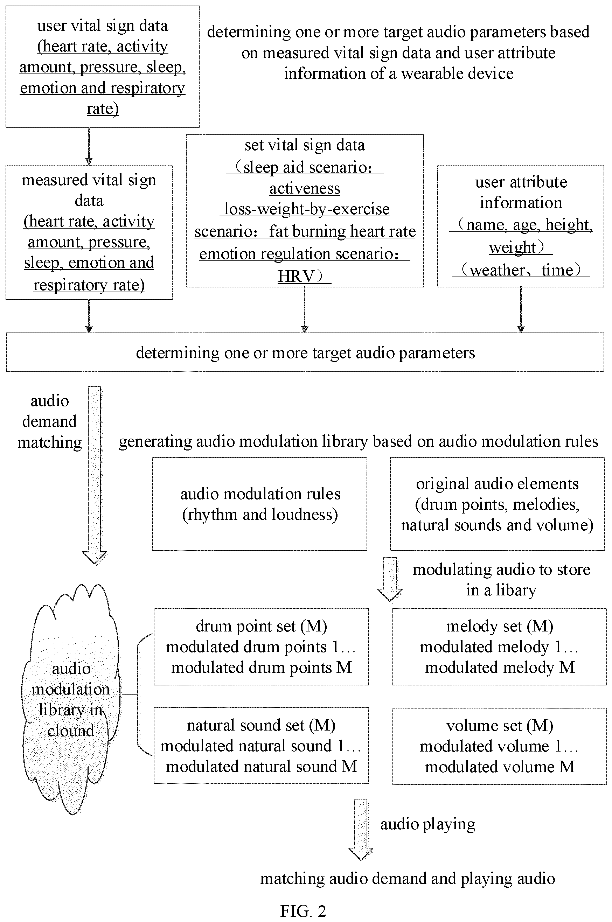

As illustrated in FIG. 2, vital sign data of a user includes one or more combinations of a heart rate, an activity amount, a pressure, sleep, an emotion and a respiratory rate.

Predicted vital sign data includes one or more combinations of the heart rate, the activity amount, the pressure, the sleep, the emotion and the respiratory rate.

For different set scenarios, target physiological parameters are different. For example, if a scenario set by the user is a sleep aid scenario, the corresponding target physiological parameter includes activeness. For example, if a scenario set by the user is a weight-loss-by-exercise scenario, the corresponding target physiological parameter includes fat burning heart rate. For example, if a scenario set by the user is an emotion regulation scenario, the corresponding target physiological parameter includes HRV

The user attribute information includes basic information of the user, which includes at least one of age, gender, height, weight or disease status. In some implementations, attributes of the user are related to the environment the user is in, and accordingly the user attribute information also includes the environment information of the environment the user is in, where the environment information includes at least one of weather or time.

Audio modulation parameters include audio loudness and rhythm. For example, target audio modulation parameters include target audio loudness and target rhythm.

Original audio elements include multiple combinations of drum points, melodies, natural sounds and loudness.

For example, as illustrated in FIG. 2, the method for audio playback is implemented in three parts. In the first part, one or more target audio parameters are determined based on measured vital sign data and the user attribute information of a wearable device, that is, natural evolution of vital signs of the user in the future is predicted based on vital sign data of the wearable device measured in real time, so as to obtain the predicted vital sign data. One or more target physiological parameters corresponding to the user scenario information are obtained based on target scenario set by the user (i.e., the user scenario information), such as a sleep aid scenario, a weight-loss-by-exercise scenario, or an emotion regulation scenario. The target physiological parameter corresponding to the sleep aid scenario optionally includes activeness, the target physiological parameter corresponding to the weight-loss-by-exercise scenario optionally includes fat burning heart rate, and the target physiological parameter corresponding to the emotion regulation scenario optionally includes HRV The user attribute information obtained via the wearable device includes basic information of the user. The basic information of the user includes at least one of the age, the gender, the height, the weight or the disease status. Based on the environment the user is in, the wearable device further obtains environment information of the environment the user is in. The environmental information includes at least one of weather or time. In this way, the one or more target audio parameters are determined based on the predicted vital sign data, the target physiological parameter and the user attribute information.

In the second part, an audio modulation library is generated based on an audio modulation rule, that is, the original audio elements are modulated based on the audio modulation rule, to generate the audio modulation library, and the audio modulation library is stored at the cloud end. The parameter for the audio modulation rule includes rhythm and loudness, and the original audio elements include drum points, melodies, natural sounds and loudness with various rhythms and loudness. In some implementations, the original audio elements are modulated into different versions of rhythms and loudness with high, medium and low proportion, to obtain modulated drum point set, melody set, natural sound set and loudness set, and the audio modulation library is generated and stored at the cloud end. It should be understood that the audio modulation library is generated in advance, that is, the audio modulation library has been generated before the matching of required audio is performed.

In the third part, the required audio is matched and then played, that is, appropriate audio is matched from the audio modulation library at the cloud end based on the one or more target audio parameters determined, and modulated audio is synthesized and played for the user, so as to finally achieve a positive guidance for the user's sleep or health.

Based on the above, the natural evolution of vital signs of the user in the future is predicted based on the vital sign data measured in real time by the wearable device, to obtain predicted vital sign data. The target physiological parameter corresponding to user scenario information is obtained based on the user scenario information of audio set by the user. The user attribute information is obtained based on the wearable device, so that the one or more target audio parameters are determined based on the predicted vital sign data, the target physiological parameter and the user attribute information. Then the appropriate audio is matched from the audio modulation library in the cloud based on the one or more target audio parameters determined, and the appropriate audio is played. Since the one or more target audio parameters are determined based on the predicted vital sign data, the target physiological parameter and the user attribute information, the target audio matched with the one or more target audio parameters can be effective for adjusting sleep or health for the user, so as to solve the technical problem of the related technologies that actual audio playback does not match the user's requirements and the adjustment effect is poor.

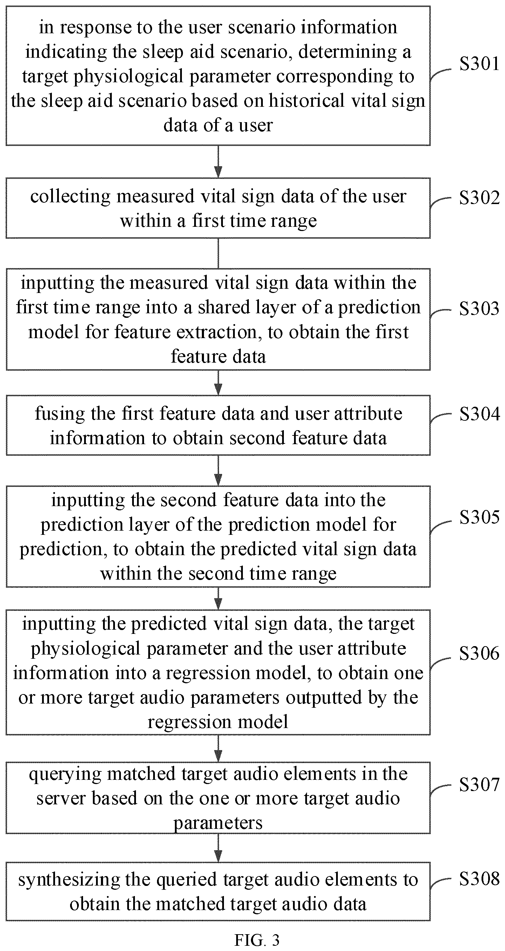

In order to illustrate the previous implementation more clearly, another method for audio playback is provided in the implementation of the disclosure. FIG. 3 is a flowchart of a method for audio playback provided in another implementation of the disclosure.

As illustrated in FIG. 3, the method for audio playback includes the following S301 to S308.

At S301, in response to user scenario information indicating the sleep aid scenario, the target physiological parameter corresponding to the sleep aid scenario is determined based on historical vital sign data of a user.

Specifically, in the case that it is determined to be the sleep aid scenario in response to an operation of the user, the target physiological parameter, which corresponds to the sleep aid scenario and is determined based on the historical vital sign data of the user, is obtained.

In some implementations, vital sign data collected in real time is stored in a database or other storage files. Vital sign data stored is taken as the historical vital sign data, and the target physiological parameter corresponding to the sleep aid scenario is determined periodically based on the historical vital sign data. Since the target physiological parameter is determined periodically based on continuous real-time vital sign data of the user, its accuracy is relatively high, and target audio data obtained can effectively regulate user's sleep. Therefore, in the case of a relatively high demand for an accuracy of the target audio, periodic execution can be adopted.

In some implementations, the vital sign data collected in real time is stored in the database or other storage files. The vital sign data stored is taken as the historical vital sign data, and in response to entering the sleep aid scenario, the target physiological parameter corresponding to the sleep aid scenario is determined based on the historical vital sign data. Since the target physiological parameter is determined immediately when entering the sleep aid scenario, the execution efficiency is high, and the target audio data is quickly obtained and sleep adjustment is performed. Therefore, in the case of a high demand for the execution efficiency of the target audio, immediate execution responsive to entering into the sleep scenario is adopted.

In the following examples of the present disclosure, the process of determining the target physiological parameter corresponding to the sleep aid scenario based on the historical vital sign data is described more specifically. The historical vital sign data of the user is obtained by the sensor mounted in the wearable device, where the historical vital sign data includes historical heart rate data and historical activity amount data. Then, the historical heart rate data and the historical activity amount data is normalized based on an average heart rate before sleep and an average activity amount in the historical vital sign data of the user, and normalized historical heart rate data $HR_{norm}$ and normalized historical activity amount *data Activity$_{norm}$* are obtained, so as to reflect the characteristics of heart rate and activity for each user individual. Then, in order to comprehensively reflect the impact of heart rate and activity amount, the normalized historical heart rate data and the normalized historical activity amount data are weightaveraged based on the following formula to determine historical activeness *Activeness.* The formula is as follows:

$$Activeness = \alpha * HR_{norm} + （1 - \alpha） * Activity_{norm.}$$

where $\alpha$ is the weight of the historical heart rate data $HR_{norm}$.

Finally, one or more target physiological parameters corresponding to the sleep aid scenario are determined according to the activeness range to which the historical activeness belongs. The activeness range is optionally divided based on the activeness before sleep of a user wearing the wearable device. In some implementations, the quartiles Q1, Q2 and Q3 are calculated by analyzing distribution of the historical activeness before sleep of the user wearing the wearable device. The quartile, also known as quartet, refers to values at positions of three segmentation points in statistics when all values sorted in an ascending order are divided into four equal parts. The activeness before sleep are discretized into three quantitative levels based on the quartiles. The activeness before sleep less than Q1 is taken as low activeness, the activeness before sleep within the range of [Q1, Q3] is taken as medium activeness, and the activeness before sleep greater than Q3 is taken as high activeness.

In some implementations, when the user is in a high activeness range, the target physiological parameter is set as a medium activeness range. When the user is in the middle activeness range, the target physiological parameter is set as a low activeness range.

At S302, measured vital sign data of the user within a first time range is collected.

In the implementation of the present disclosure, the measured vital sign data includes monitored data of the heart rate and monitored data of the activity amount.

The execution process of S 101 in the above implementations can be referred to for other execution process of this procedure, which will not be repeated herein.

At S303, the measured vital sign data within the first time range is inputted into a shared layer of a prediction model for a feature extraction, to obtain first feature data.

The measured vital sign data includes the monitored data of the heart rate and the monitored data of the activity amount.

The monitored data of the heart rate within the first time range is inputted into the shared layer of the prediction model for feature extraction, to obtain the first feature data corresponding to the heart rate. The monitored data of the activity amount within the first time range is inputted into the shared layer of the prediction model for feature extraction, to obtain the first feature data corresponding to the activity amount.

It should be noted that, the monitored data of heart rate and the monitored data of activity amount are inputted into the same shared layer of the prediction model, or the monitored data of heart rate and the monitored data of activity amount are inputted into different shared layers of the prediction model.

At S304, the first feature data and user attribute information are fused to obtain second feature data.

The user attribute information includes basic information of the user. The basic information of the user includes at least one of age, gender, height, weight or disease status. In some implementations, attributes of the user are related to the environment the user is in, and accordingly the user attribute information also includes environment information of the environment the user is in, where the environment information includes at least one of weather or time.

In some implementations, the first feature data corresponding to the heart rate and the first feature data corresponding to the activity amount obtained in the previous procedure are spliced with the user attribute information to obtain the second feature data. Since the second feature data is obtained by directly splicing the first feature data and user attribute information, the execution efficiency is high, and the second feature data is obtained quickly. Therefore, when the predicted vital sign data needs to be obtained quickly, the manner of direct splicing is adopted.

In some implementations, the first feature data corresponding to the heart rate and the first feature data corresponding to the activity amount obtained in the previous procedure are weighted-fused with the user attribute information to obtain the second feature data. Weights for the first feature data corresponding to the heart rate, the first feature data corresponding to the activity amount and user attribute information can be pre-configured, and there is a certain correlation between the weights and the degree of influence on the predicted vital sign data. Those skilled in the art know that the specific values of the weights may be obtained by a limited number of tests. Among the different values of the weights, the values which bring the most accurate value of the predicted vital sign data is used for the weighted fusion of the first feature data corresponding to heart rate, the first feature data corresponding to activity and user attribute information. Since the second feature data is obtained by a weighted fusion of the first feature data and user attribute information and the weights need to be obtained by a plurality of experiments, it has relatively high accuracy and brings the most accurate value for the predicted vital sign data. Therefore, the manner of weighted fusion is used in the case that accurate prediction of the vital sign data is required.

At S305, the second feature data is inputted into the prediction layer of the prediction model for prediction, to obtain the predicted vital sign data within the second time range.

In some implementations, the second feature data is inputted into the prediction layer of the prediction model corresponding to the heart rate for heart rate prediction, so as to obtain the prediction data of the heart rate, and the second feature data is inputted into the prediction layer of the prediction model corresponding to the activity amount for activity amount prediction, so as to obtain the prediction data of the activity amount.

In the implementation of the present disclosure, the measured vital sign data includes the monitored data of heart rate and the monitored data of activity amount.

At S306, the predicted vital sign data, the target physiological parameter and the user attribute information are inputted into a regression model, to obtain one or more target audio parameters outputted by the regression model.

In some implementations, the regression model is a trained model and has learned a mapping relationship between the predicted vital sign data, the target physiological parameter and user attribute information with the one or more target audio parameters.

The user attribute information includes the basic information of the user, which includes at least one of the age, the gender, the height, the weight or the disease status.

In some implementations, user's attributes are related to the environment the user is in, so the user attribute information further includes the environment information of the environment the user is in, and the environment information includes at least one of weather or time. For example, the weather is sunny, cloudy, etc., and the time is related to the target moment, specifically at least part of the day, month, hour, minute, or second. The time may include only hour, minute, and second. The choice of time granularity is determined according to the prediction accuracy. Generally, there is a periodic change in emotion of the user within a day. For example, in the morning, the psychological pressure is usually low and gradually becomes intense over time, and the pressure has a decline trend near noon. Similarly, due to different activities carried out by the user, different months or seasons within a year show different characteristics. Therefore, in order to accurately predict the user's demand, it is necessary to select the appropriate time granularity according to the prediction accuracy. In the case of a relatively high demand for prediction accuracy, combinations of more time granularities are selected, such as day and month, as well as hour, minute and second. In the case of a relatively low demand for prediction accuracy, a combination with less time granularities are selected, such as hour and minute.

The one or more target audio parameters include target audio loudness and target rhythm. Loudness of audio refers to the intensity of the sound sensed by the ear, and it is a subjective perception of the volume of the sound. Rhythm refers to a regular mutation that accompanies rhythm in natural, social and human activities.

It should be understood that, before the predicted vital sign data, the target physiological parameter and the user attribute information are inputted into the regression model, a feature engineering processing needs to be performed on the predicted vital sign data, the target physiological parameter and the user attribute information, so as to convert them into data that is suitable for the regression model to process.

The regression model has been obtained by the process of model training before the predicted vital sign data, the target physiological parameter and the user attribute information are inputted into the regression model. In some implementations, a training process is performed before the wearable device is delivered from the factory. For example, the regression model is trained by vital sign data of test users collected offline, and the regression model is loaded into the wearable device after it is trained. In some implementations, during usage of the user, the vital sign data of the user is recorded, and one or more historical data samples are generated based on the audio selected by the user's audio selection operations, and the regression model is trained by using this data to obtain a regression model with a better adjustment effect.

At S307, matched target audio elements are queried in the server based on the one or more target audio parameters.

In the implementation of the present disclosure, audio elements are modulated and classified, and an audio modulation library is generated and stored in the server. The audio elements include a plurality of combinations of drum points, melodies, natural sounds and loudness.

In some implementations, the matched target audio elements are queried in the server based on the one or more target audio parameters, i.e., the target audio loudness and the target rhythm. Audio elements with a plurality of rhythms and loudness are stored in the server. These audio elements are obtained in advance by processing the audio in an audio set. In some implementations, the audio in the audio set is modulated according to an audio modulation rule. For example, the drum points and the melodies are main parts of the audio for adjusting the emotion of the user and rhythm. The natural sound and loudness are used as auxiliary parts for creating the environment atmosphere. Therefore, the drum points, the melodies, the natural sounds and loudness in the audio set are modulated into different rhythm and loudness versions with high, medium and low modulation proportion according to the audio modulation rule, to obtain modulated drum point set, melody set, natural sound set and loudness set, and the audio modulation library is generated and stored in the server.

At S308, the queried target audio elements are synthesized to obtain the matched target audio data.

In this procedure, by obtaining the target audio data, the wearable device plays the target audio for the user.

In some implementations, the matched target audio elements queried in the server are synthesized to obtain the target audio data.

In some implementations, the drum points, the melodies, the natural sounds and the loudness having the target audio loudness and the target rhythm that are obtained in the previous procedure are input into different audio tracks for synthetization, so as to obtain the target audio for play.

In S307, the audio elements are modulated and classified in advance, and the audio modulation library is generated and stored in the server. In S308, the target audio elements matching the one or more target audio parameters are queried in the audio modulation library based on the one or more target audio parameters obtained in S306. Then the queried target audio elements are synthesized to obtain the target audio data.

Based on the above, the measured vital sign data within the first time range is collected. The vital sign prediction is performed based on the measured vital sign data within the first time range to obtain the predicted vital sign data of the user within the second time range after the first time range. The one or more target audio parameters are determined based on the predicted vital sign data, the target physiological parameter and the user attribute information. And the matched target audio data is obtained based on the one or more target audio parameters. Since the one or more target audio parameters are determined based on the predicted vital sign data, the target physiological parameter and the user attribute information, the target audio data matched with the one or more target audio parameters can be effective for adjusting sleep or health for the user, so as to solve the technical problem of the related technologies that actual audio playback does not match requirements of the user and the adjustment effect is poor.

In order to illustrate the acquisition of the predicted vital sign data more clearly, an example is given herein for illustration.

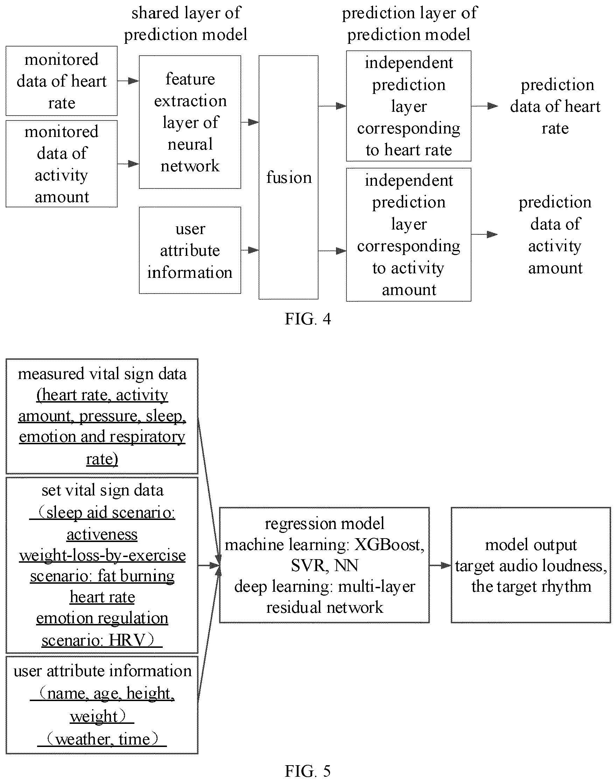

For example, the prediction model shown in FIG. 4 fully captures change rules of vital sign data such as a heart rate, activity amount and a pressure over time by using multi-task learning technology in deep learning, so as to perform the vital sign prediction. Taking the heart rate and the activity amount as an example, the monitored data of the heart rate and the monitored data of the activity amount are inputted into a neural network feature extraction layer of the shared layer of the prediction model, to extract first feature data of vital sign data, so that the first feature data corresponding to the heart rate and the first feature data corresponding to the activity amount are obtained. The monitored data of the heart rate and the monitored data of the activity amount are obtained based on the wearable device. Then the first feature data and user attribute information are fused, that is, the first feature data corresponding to the heart rate and the first feature data corresponding to the activity amount are spliced with the user attribute information respectively, to obtain second feature data corresponding to the heart rate and the second feature data corresponding to the activity amount. The user attribute information includes basic information of the user. The basic information of the user includes at least one of age, gender, height, weight or disease status. In some implementations, the user attribute information further includes environment information of the environment the user is in. The environment information includes at least one of weather or time. Finally, the second feature data is inputted into the prediction layer of the prediction model for prediction, that is, the second feature data corresponding to the heart rate and the second feature data corresponding to activity amount are respectively inputted into the independent prediction layer corresponding to the heart rate and the independent prediction layer corresponding to activity amount for prediction, so as to obtain the prediction data of the heart rate and the prediction data of the activity amount.

It should be noted that, shared layers of the prediction model to which the monitored data of the heart rate and the monitored data of the activity amount are inputted respectively are the same or different. In this implementation of the present disclosure, the monitored data of the heart rate and the monitored data of the activity amount are inputted into a same shared layer of the prediction model.

In some implementations, the first feature data corresponding to the heart rate and the first feature data corresponding to the activity amount are spliced with the user attribute information respectively to obtain the second feature data.

In some implementations, the first feature data corresponding to the heart rate and the first feature data corresponding to the activity amount are weighted-fused with the user attribute information to obtain the second feature data.

Based on the above, the monitored data of the heart rate and the monitored data of the activity amount are inputted into the neural network feature extraction layer of the shared layer of the prediction model, and the first feature data of the vital sign data is extracted, so that the first feature data corresponding to the heart rate and the first feature data corresponding to the activity amount are obtained. Then the first feature data corresponding to the heart rate and the first feature data corresponding to the activity amount are spliced with the user attribute information respectively, to obtain the second feature data corresponding to the heart rate and the second feature data corresponding to the activity amount. Finally, the second feature data corresponding to the heart rate and the second feature data corresponding to the activity amount are respectively inputted into the independent prediction layer corresponding to the heart rate and the independent prediction layer corresponding to the activity amount for prediction, so as to obtain the prediction data of the heart rate and the prediction data of the activity amount. Therefore, change rules of heart rate and activity over time are fully captured, and the corresponding predicted vital sign data are obtained.

In order to illustrate determination of the one or more target audio parameters more clearly, an example is given for illustration.

For example, as illustrated in FIG. 5, a regression model is used. Predicted vital sign data, target physiological parameter and user attribute information are inputted into the regression model, to obtain one or more target audio parameters outputted by the regression model. The predicted vital sign data includes one or more combinations of a heart rate, an activity amount, a pressure, sleep, an emotion and a respiratory rate.

The user attribute information includes basic information of the user, which includes at least one of age, gender, height, weight or disease status. In some implementations, attributes of the user are related to an environment the user is in, so the user attribute information also includes environment information of the environment the user is in, and the environment information includes at least one of weather or time.

The one or more target audio parameters include target audio loudness and target rhythm.

In some implementations, the regression model is a trained model and has learned a mapping relationship between the predicted vital sign data, the target physiological parameter and the user attribute information with the one or more target audio parameters. The regression model is a machine learning regression model such as a XGBoost (extreme gradient boosting), a SVR (support vector regression), a NN (neural network), or a deep learning regression model such as a multi-layer residual network.

In the implementation of the present disclosure, it is necessary to perform a feature engineering processing on the predicted vital sign data, the target physiological parameter and the user attribute information in advance, so as to convert them into data that is suitable for regression model to process.

In some implementations, the gender in the basic user information and the weather in the environment information are used as categorical data, using single-hot coding. Others are numerical data, which are processed by min-max normalization or gaussian standardization.

Further, data processed by the feature engineering processing, i.e., the predicted vital sign data, the target physiological parameter and the user attribute information are inputted into a regression model, to obtain the one or more target audio parameters outputted by the regression model, i.e., the target audio loudness and the target rhythm.

Based on the above, the data processed by the feature engineering processing, i.e., the predicted vital sign data, the target physiological parameter and the user attribute information are inputted into the regression model, to obtain the one or more target audio parameters outputted by the regression model. Thus, the target audio data matched with the one or more target audio parameters can be effective for adjusting sleep or health for the user, so as to solve the technical problem of the related technologies that actual audio playback does not match the user's requirements and the adjustment effect is poor.

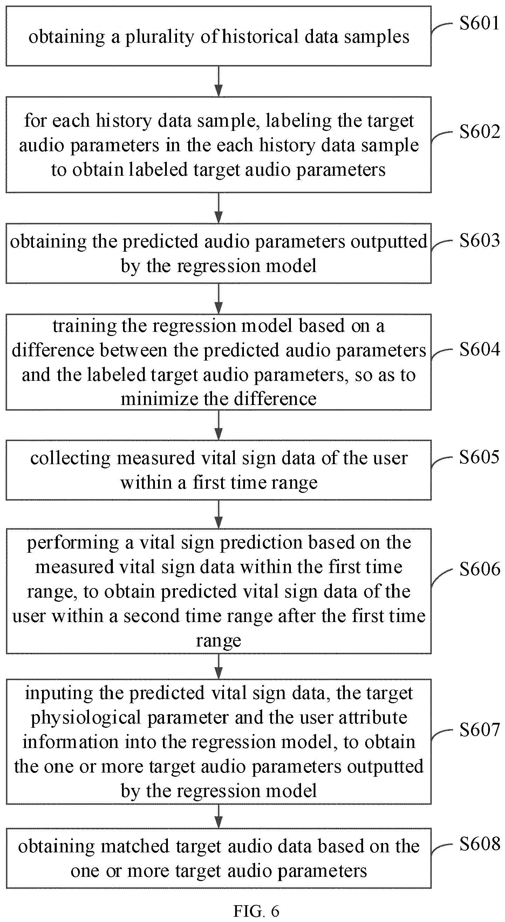

In order to make the one or more target audio parameters outputted by the regression model accurately, another method for audio playback is provided in one implementation of the disclosure. FIG. 6 is a flowchart of a method for audio playback provided in another implementation of the disclosure.

As illustrated in FIG. 6, the method for audio playback includes the following S601 to S608.

At S601, a plurality of historical data samples are obtained.

In the implementations of the present disclosure, the historical data sample includes user vital sign data, target physiological parameter, user attribute information and one or more target audio parameters. The user vital sign data includes measured vital sign data and predicted vital sign data. The user vital sign data includes one or more combinations of a heart rate, an activity amount, a pressure data, sleep data, emotion data and a respiratory rate. The user attribute information includes basic information of the user, the basic information of the user includes at least one of age, gender, height, weight or disease status. The user attribute information also includes environment information of the environment that the user lives in, and the environment information includes at least one of weather or time.

In some implementations, the target audio parameters include target audio loudness and target rhythm.

In some implementations, the historical data sample is collected online. For example, data related to a user wearing the wearable device, that is collected online by using web crawler technologies, is taken as the historical data sample.

In some implementations, the historical data sample is collected offline. For example, the data related to the user wearing the wearable device, that is collected offline by participating in the test, is taken as the historical data sample, etc.

At S602, for each history data sample, the target audio parameters in the each history data sample are labeled to obtain labeled target audio parameters.

In the implementation of the disclosure, manual labeling is used to improve a training effect of the model.

In some implementations, for each history data sample including the predicted vital sign data, the target physiological parameter corresponding to user scenario information, the user attribute information and the target audio parameters, the target audio parameters including the target audio loudness and the target rhythm are labeled, to obtain the labeled target audio parameters.

At S603, the predicted audio parameters outputted by the regression model are obtained.

In the implementation of the present disclosure, it is necessary to perform a feature engineering processing on the predicted vital sign data, the target physiological parameter and the user attribute information in advance, so as to convert them into data that is suitable for a regression model to process.

In some implementations, the gender in the user's basic information and the weather in the environment information are categorical data and are processed by single-hot coding, and other data is numerical data and is processed by min-max normalization or by gaussian normalization.

In the implementation of the present disclosure, the regression model is a machine learning regression model such as a XGBoost, a SVR, a NN, or the regression model is a deep learning regression model such as a multi-layer residual network, or the like.

Further, data processed by the feature engineering processing is inputted into the regression model, to obtain the target audio parameters outputted by the regression model.

At S604, the regression model is trained based on a difference between the predicted audio parameters and the labeled target audio parameters, so as to minimize the difference.

In the implementation of the present disclosure, after the predicted audio parameters outputted by the regression model are obtained, the predicted audio parameters are compared with the labeled target audio parameters to determine the difference between the predicted audio parameters and the labeled target audio parameters. Parameters of the regression model are adjusted according to the difference so as to minimize the difference, thereby obtaining a trained regression model that has learned the mapping relationship between the predicted vital sign data, the target physiological parameter and the user attribute information with the target audio parameters.

It should be understood that, in some implementations, this training process is performed before the wearable device is delivered from a factory. For example, the regression model is trained by the user vital sign data collected and tested offline. The regression model is loaded into the wearable device after it is trained.

In some implementations, the vital sign data of the user is recorded, and one or more historical data samples are generated based on the audio selected by the user's audio selection operation, and the regression model is trained by using this data to obtain the regression model with a better adjustment effect.

In some implementations, this training process is performed during the usage of the wearable device. For example, the user vital sign data collected online in the previous audio playback process, and the loudness and rhythm of the audio selected by the user's operation are used as labels to train the regression model, to obtain the trained regression model that has learned the mapping relationship between the predicted vital sign data, the target physiological parameter and the user attribute information with the target audio parameters. The user vital sign data and the loudness and the rhythm of the audio selected by the user's operation are recorded in real time during the user's usage. The regression model is constantly trained to obtain the regression model with a better adjustment effect.

In the implementation of the present disclosure, one or more historical data samples are obtained from the server during the user's usage, and the historical data samples are used to train the regression model to obtain the regression model with a better adjustment effect.

At S605, measured vital sign data of the user within a first time range is collected.

At S606, a vital sign prediction is performed based on the measured vital sign data within the first time range, to obtain predicted vital sign data of the user within a second time range after the first time range.

At S607, the predicted vital sign data, the target physiological parameter and the user attribute information are inputted into the regression model, to obtain the one or more target audio parameters outputted by the regression model.

At S608, matched target audio data is obtained based on the one or more target audio parameters.

It should be noted that the execution process of S605 to S608 refer to the execution process of S101 to S104, which will not be repeated herein.

Based on the above, a plurality of history data samples are obtained, and the target audio parameters in the history data sample are labeled for each history data sample, to obtain labeled target audio parameters. After the predicted audio parameters outputted by the regression model are obtained, the regression model is trained based on a difference between the predicted audio parameters and the labeled target audio parameters, to minimize the difference.

Therefore, the regression model is trained, and the regression model has learned the mapping relationship between the predicted vital sign data, the target physiological parameter and the user attribute information with the target audio parameters, so that the regression model accurately outputs the target audio parameters.

In order to achieve the above implementations, an apparatus for audio playback is further provided in the disclosure.

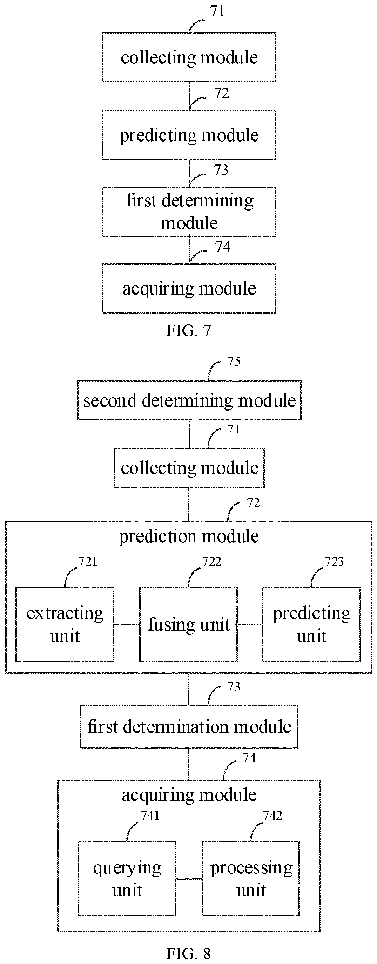

FIG. 7 is a structure diagram of an apparatus for audio playback provided in the implementation of the present disclosure.

As illustrated in FIG. 7, the apparatus for audio playback includes: a collecting module 71, a predicting module 72, a first determining module 73 and an acquiring module 74.

The collecting module 71 is configured to obtain measured vital sign data of a user within a first time range.

The predicting module 72 is configured to perform a vital sign prediction based on the measured vital sign data within the first time range, to obtain predicted vital sign data of the user within a second time range after the first time range.

The first determining module 73 is configured to determine one or more target audio parameters based on the predicted vital sign data and user attribute information of the user.

Specifically, the first determination module 73 is configured to input the predicted vital sign data, target physiological parameter and the user attribute information into a regression model, to obtain the one or more target audio parameters outputted by the regression model.

The one or more target audio parameters include target audio loudness and target rhythm. The user attribute information includes basic information of the user, the basic information of the user includes at least one of age, gender, height, weight or disease status. In some implementations, the user attribute information also includes environment information of the environment the user is in, and the environment information includes at least one of weather or time.

The acquiring module 74 is configured to obtain matched target audio data based on the one or more target audio parameters.

Further, the prediction module 72 includes an extracting unit 721, a fusing unit 722, and a predicting unit 723.

The extracting unit 721 is configured to perform feature extraction processing on the measured vital sign data within the first time range, to obtain first feature data.

The fusing unit 722 is configured to fuse the first feature data and the user attribute information, to obtain second feature data.

The predicting unit 723 is configured to perform prediction based on the second feature data, to obtain the predicted vital sign data within the second time range.

Furthermore, vital sign data of the user includes monitored data of heart rate and the monitored data of activity amount.

In some implementations, the extraction unit 721 is configured to: input the monitored data of the heart rate within the first time range into a shared layer of a prediction model for feature extraction, to obtain the first feature data corresponding to the heart rate; and input the monitored data of the activity amount within the first time range into the shared layer of the prediction model for feature extraction, to obtain the first feature data corresponding to the activity amount.

Furthermore, the predicted vital sign data includes prediction data of the heart rate and prediction data of the activity amount.

The predicting unit 723 is configured to: input the second feature data into a prediction layer of the prediction model corresponding to the heart rate to predict the heart rate, so as to obtain the prediction data of the heart rate; and input the second feature data into the prediction layer of the prediction model corresponding to the activity amount to predict the activity amount, so as to obtain the prediction data of the activity amount.

Specifically, the first determination module 73 is configured to input the predicted vital sign data, the target physiological parameter and the user attribute information into the regression model, to obtain the one or more target audio parameters outputted by the regression model. The one or more target audio parameters include the target audio loudness and the target rhythm.

In some implementations, the acquiring module 74 includes a querying unit 741 and a processing unit 742.

The querying unit 741 is configured to query matched audio elements on the server based on the target audio loudness and the target rhythm. Audio elements include a plurality of combinations of drum points, melodies, natural sounds and loudness.

The processing unit 742 is configured to synthesize queried audio elements to obtain target audio and play it.

In some implementations, the querying unit 741 is configured to send target audio modulation parameters to a server, so as to enable the server to query target audio data matching the target audio modulation parameters. The processing unit 742 is configured to obtain the target audio data sent by the server.

It needs to be noted that the foregoing explanation of the implementation of the method for audio playback is also applied to an apparatus for audio playback in the implementation, which is not be repeated herein.

Based on the above implementations, the present disclosure further provides a possible implementation of an apparatus for audio playback. FIG. 8 is a structural diagram of an apparatus for audio playback provided in another implementation of the present disclosure. On the basis of the previous implementation, the apparatus for audio playback further includes a second determining module 75.

The second determining module 75 is configured to obtain historical heart rate data and historical activity amount data in the case that the scenario is set as a sleep aid scenario; determine historical activeness based on a weighted sum of the historical heart rate data and the historical activity amount data; determine the target physiological parameter corresponding to the sleep aid scenario, based on the activeness range to which the historical activeness belongs.

In some implementations, the target physiological parameter includes the activeness. The second determining module 75 is configured to obtain historical heart rate data and historical activity data of the user within the third time range corresponding to the first time range; determine the historical activeness of the user based on the historical heart rate data and the historical activity data of the user; and determine the target activeness of the user according to the historical activeness of the user. The target activeness is lower than the historical activeness.

In the implementation of the present disclosure, measured vital sign data of a user within a first time range is obtained; a vital sign prediction is performed based on the measured vital sign data within the first time range, to obtain predicted vital sign data of the user within a second time range after the first time range; one or more target audio parameters are determined based on the predicted vital sign data and user attribute information of the user; and matched target audio data is obtained based on the one or more target audio parameters. Since the one or more target audio parameters are determined based on the predicted vital sign data, the target physiological parameter and the user attribute information, the target audio data matched with the one or more target audio parameters can be effective for adjusting sleep or health for the user, so as to solve the technical problem the related technologies that actual audio playback does not match requirements of the user and the adjustment effect is poor.

In order to achieve the above implementation, a wearable device is further provided in the disclosure. The wearable device includes at least one processor; and a memory communicatively connected to the at least one processor; the memory is stored with instructions executable by the at least one processor, and the instructions are performed by the at least one processor, so that the at least one processor performs the method for audio playback provided in the above any implementation of the disclosure.

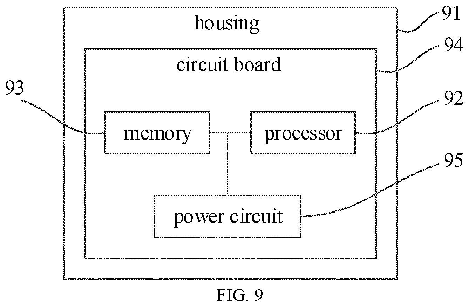

FIG. 9 is a structural diagram of a wearable device provided in one implementation of the present disclosure, and flow of the implementations shown in FIGS. 1 to 8 in the present disclosure are realized.

As shown in FIG. 9, the wearable device includes a housing 91, a processor 92, a memory 93, a circuit board 94 and a power circuit 95. The circuit board 94 is disposed inside the space enclosed by the housing 91, and the processor 92 and memory 93 are disposed on the circuit board 94. The power supply circuit 95 is configured to supply power for each circuit or device of the wearable device. The memory 93 is configured to store executable program code. The processor 92 executes the program corresponding to the executable program code by reading the executable program code stored in the memory 93, and is used to execute the method for audio playback described in any of the aforementioned implementations.

The specific execution process of the above procedures by the processor 92 and the further execution procedures by the processor 92 by running executable program code are described in the implementations shown in FIGS. 1 to 8 of the present disclosure, which are not be repeated here.

In order to achieve the above implementation, a non-transitory computer readable storage medium stored with computer instructions is further provided, the computer instructions are configured to perform the method for audio playback as described in the above any implementation of the disclosure by a wearable device.

In the disclosure, descriptions with reference to terms "one implementation", "some implementations", "example", "specific example" or "some examples" mean specific features, structures, materials or features described in combination with the implementation or example are included in at least one implementation or example of the disclosure. The schematic representations of the above terms do not have to be the same implementation or example. Moreover, specific features, structures, materials or features described are combined in one or more implementations or examples in a suitable manner. Furthermore, implementations or examples described in the specification, as well as features of implementations or examples, are combined without conflicting with each other.

In addition, the terms "first" and "second" are only for describing purposes and are not to be construed as indicating or implying relative importance or implicitly indicating the number of technical features indicated. Thus, features limiting "first" and "second" explicitly or implicitly include at least one of the features. In the description of the disclosure, "a plurality of" means at least two, for example, two, three, unless otherwise expressly and specifically stated.

Any process or method described in the flowchart or otherwise described herein is understood as representing one or more modules, segments, or portions of codes of executable instructions for implementing the blocks of a customized logical function or process, and the scope of the implementations of the present disclosure includes additional implementations, the functions are executed not in the sequence shown or discussed, including in a substantially simultaneous manner or in a reverse sequence, which will be appreciated by those skilled in the art the implementations of the disclosure belong to.

The logics and/or blocks represented in the flowchart or described in other ways herein, for example, are considered as an ordered list of executable instructions configured to implement logic functions, which are specifically implemented in any computer readable medium for use by a system, an apparatus or a device for executing instructions (such as a system based on a computer, a system including a processor, or other systems that obtain and perform instructions from a system, an apparatus or a device for performing instructions) or in combination with the system, the apparatus or the device for performing instructions. A "computer readable medium" in the disclosure is an apparatus that contains, stores, communicates, propagates or transmits a program for use by a system, an apparatus or a device for executing instructions or in combination with the system, the apparatus or the device for executing instructions. A more specific example (a non-exhaustive list) of a computer readable medium includes the followings: an electronic connector (an electronic apparatus) with one or more cables, a portable computer disk box (a magnetic device), a random access memory (RAM), a read-only memory (ROM), an electrically programmable read-only memory (an EPROM or a flash memory), an optical fiber apparatus, and a portable optical disk read-only memory (CDROM). In addition, a computer readable medium even is paper or other suitable medium on which a program is printed, since paper or other medium is optically scanned, and then edited, interpreted or processed in other suitable ways if necessary to obtain a program electronically and store it in a computer memory.

It should be understood that all parts of the present disclosure are implemented with a hardware, a software, a firmware and their combination. In the above implementation, a plurality of blocks or methods are stored in a memory and implemented by a software or a firmware executed by a suitable system for executing instructions. For example, if implemented with a hardware, they are implemented by any of the following technologies or their combinations known in the art as in another implementation: a discrete logic circuit with logic gate circuits configured to achieve logic functions on data signals, a special integrated circuit with appropriate combined logic gate circuits, a programmable gate array (PGA), a field programmable gate array (FPGA), etc.

Those skilled in the art understand that all or part of blocks in the above method implementations are implemented by instructing relevant hardware by computer programs. The programs are stored in a computer readable storage medium, and the programs include one of blocks of the method implementations or their combination when executed.

In addition, functional units in the implementations of the disclosure are integrated in one processing module, or each of the units is physically existed alone, or two or more units are integrated in one module. The integrated module is achieved by a form of a hardware, and also is achieved by a form of a software functional module. The integrated module is stored in a computer readable storage medium when it is implemented in a form of a software functional module and sold or used as an independent product.

The above storage medium is a read-only memory, a magnetic disk or an optical disk. Even though implementations of the disclosure have been illustrated and described above, it can be understood that the above implementations are exemplary and may not be construed as a limitation of the present disclosure, and changes, modifications, substitutions and alterations are made to the above implementations within the scope of the disclosure.

Those skilled in the art understand that all or part of processes in the above implementations are implemented by instructing relevant hardware by computer programs. The programs are stored in a computer readable storage medium, and the programs include processes of the above implementations when executed. The storage medium is a magnetic disk, an optical disk, a read-only memory (ROM) or a random access memory (RAM), etc.

The above are only specific implementations of the disclosure, however, the protection scope of the present disclosure is not limited here, and those skilled in the art easily think of any changes or substitutions within the technical scope of the present disclosure, which shall be within the protection scope of the disclosure. Therefore, the protection scope of the disclosure shall be subject to the protection scope of the claims.

**Claims**

1. A method for audio playback, comprising:

   obtaining measured vital sign data of a user within a first time range;
   performing a vital sign prediction based on the measured vital sign data within the first time range, to obtain predicted vital sign data of the user within a second time range after the first time range;
   determining one or more target audio parameters based on the predicted vital sign data and user attribute information of the user; and
   obtaining matched target audio data based on the one or more target audio parameters.

2. The method of claim 1, wherein obtaining the measured vital sign data of the user within the first time range comprises:
   obtaining the measured vital sign data of the user within the first time range based on user scenario information, wherein the user scenario information is configured to indicate health requirements of the user.

3. The method of claim 2, wherein obtaining the measured vital sign data of the user within the first time range based on user scenario information comprises:

in response to the user scenario information indicating a sleep aid scenario or a focus scenario, obtaining heart rate data and activity data of the user within the first time range; or
in response to the user scenario information indicating a weight loss scenario, obtaining at least one of activity data, motion data, heart rate data, or positioning data of the user within the first time range; or
in response to the user scenario information indicating an emotion regulation scenario, obtaining heart rate variability of the user within the first time range.

4. The method of any one of claims 1 to 3, wherein performing the vital sign prediction based on the measured vital sign data within the first time range, to obtain the predicted vital sign data of the user within the second time range after the first time range comprises:

performing feature extraction processing on the measured vital sign data within the first time range, to obtain first feature data;
performing fusing processing on the first feature data and the user attribute information, to obtain second feature data; and
performing the vital sign prediction based on the second feature data, to obtain the predicted vital sign data within the second time range.

5. The method of any one of claims 1 to 4, wherein determining one or more target audio parameters based on the predicted vital sign data and user attribute information of the user comprises:

determining a target physiological parameter of the user; and
determining the one or more target audio parameters based on the predicted vital sign data, the target physiological parameter and the user attribute information.

6. The method of claim 5, wherein determining the target physiological parameter of the user comprises:
determining the target physiological parameter of the user based on at least one of user scenario information or historical vital sign data of the user.

7. The method of claim 5 or 6, wherein determining the target physiological parameter of the user comprises:

in response to the user scenario information indicating the sleep aid scenario or the focus scenario, determining target activeness of the user; or
in response to the user scenario information indicating the weight loss scenario, determining at least one of target activity, target activity amount or target heart rate of the user; or
in response to the user scenario information indicating the emotion regulation scenario, determining heart rate variability of the user.

8. The method of any one of claims 5 to 7, wherein, the target physiological parameter comprises activeness; determining the target physiological parameter of the user comprises:

obtaining historical heart rate data and historical activity data of the user within a third time range corresponding to the first time range;
determining the historical activeness of the user based on the historical heart rate data and the historical activity data of the user; and
determining the target activeness of the user based on the historical activeness of the user, wherein the target activeness is less than the historical activeness.

9. The method of any one of claims 5 to 8, wherein determining one or more target audio parameters based on the predicted vital sign data and user attribute information of the user comprises:
inputting the predicted vital sign data, the target physiological parameter and the user attribute information into a regression model, to obtain the one or more target audio parameters outputted by the regression model, wherein the one or more target audio parameters comprise target audio loudness and target rhythm.

**10.** The method of any one of claims 1 to 9, wherein, the one or more target audio parameters comprise a target audio modulation parameter;

obtaining matched target audio data based on the one or more target audio parameters comprises:

sending the target audio modulation parameter to a server, so as to enable the server to query target audio data matching the target audio modulation parameter; and
obtaining the target audio data sent by the server.

**11.** A method for audio playback, comprising:

determining a target physiological parameter of a user based on user scenario information, wherein the user scenario information is configured to indicate health requirements of the user;
determining one or more target audio parameters based on the target physiological parameter of the user; and
obtaining matched target audio data based on the one or more target audio parameters.

**12.** The method of claim 11, wherein determining the target physiological parameter of the user based on the user scenario information comprising:

in response to the user scenario information indicating a sleep aid scenario or a focus scenario, determining target activeness of the user; or
in response to the user scenario information indicating a weight loss scenario, determining at least one of target activeness, target activity amount and target heart rate of the user; or
in response to the user scenario information indicating an emotion regulation scenario, determining heart rate variability of the user.

**13.** The method of claim 11 or 12, wherein determining the one or more target audio parameters based on the target physiological parameter of the user comprises:

obtaining measured vital sign data of the user within a first time range based on the user scenario information;
performing a vital sign prediction based on the measured vital sign data within the first time range, to obtain predicted vital sign data of the user within a second time range after the first time range;
determining the one or more target audio parameters based on the predicted vital sign data, the target physiological parameter and user attribute information.

**14.** The method of claim 13, wherein obtaining the measured vital sign data of the user within the first time range based on the user scenario information comprises:

in response to the user scenario information indicating a sleep aid scenario or a focus scenario, obtaining heart rate data and activity data of the user within the first time range; or
in response to the user scenario information indicating a weight loss scenario, obtaining at least one of activity data, motion data, heart rate data, or positioning data of the user within the first time range; or
in response to the user scenario information indicating an emotion regulation scenario, obtaining heart rate variability of the user within the first time range.

**15.** The method of claim 13 or 14, wherein performing the vital sign prediction based on the measured vital sign data within the first time range, to obtain the predicted vital sign data of the user within the second time range after the first time range comprises:

performing feature extraction processing on the measured vital sign data within the first time range, to obtain first feature data;
performing fusing processing on the first feature data and the user attribute information, to obtain second feature data; and
performing the vital sign prediction based on the second feature data, to obtain the predicted vital sign data within the second time range.

**16.** The method of any one of claims 13 to 15, wherein determining the one or more target audio parameters based on the predicted vital sign data, the target physiological parameter and the user attribute information comprises:
inputting the predicted vital sign data, the target physiological parameter and the user attribute information into a regression model, to obtain the one or more target audio parameters outputted by the regression model, wherein the one or more target audio parameters comprise target audio loudness and target rhythm.

17. The method of any one of claims 11 to 16, wherein, the one or more target audio parameters comprise a target audio modulation parameter;
obtaining matched target audio data based on the one or more target audio parameters comprises:

sending the target audio modulation parameter to a server, so as to enable the server to query target audio data matching the target audio modulation parameter; and
obtaining the target audio data sent by the server.

18. An apparatus for audio playback, comprising:

a collecting module, configured to obtain measured vital sign data of a user within a first time range;
a predicting module, configured to perform a vital sign prediction based on the measured vital sign data within the first time range, to obtain predicted vital sign data of the user within a second time range after the first time range; and
a first determining module, configured to determine one or more target audio parameters based on the predicted vital sign data and user attribute information of the user; and
an acquiring module, configured to obtain matched target audio data based on the one or more target audio parameters.

19. The apparatus of claim 18, wherein the collecting module is configured to:
obtain the measured vital sign data of the user within the first time range based on user scenario information, wherein the user scenario information is configured to indicate health requirements of the user.

20. The apparatus of claim 19, wherein the collecting module is configured to:

in response to the user scenario information indicating a sleep aid scenario or a focus scenario, obtain heart rate data and activity data of the user within the first time range; or
in response to the user scenario information indicating a weight loss scenario, obtain at least one of activity data, motion data, heart rate data or positioning data of the user within the first time range; or
in response to the user scenario information indicating an emotion regulation scenario, obtain heart rate variability of the user within the first time range.

21. The apparatus of any of claims 18 to 20, wherein the predicting module comprises:

an extracting unit, configured to perform feature extraction processing on the measured vital sign data within the first time range, to obtain first feature data;
a fusing unit, configured to perform fusing processing on the first feature data and the user attribute information, to obtain second feature data; and
a predicting unit, configured to perform prediction based on the second feature data, to obtain the predicted vital sign data within the second time range.

22. The apparatus of any of claims 18 to 21, wherein the first determining module is further configured to:

determine a target physiological parameter of the user; and
determine the one or more target audio parameters based on the predicted vital sign data, the target physiological parameter and the user attribute information.

23. The apparatus of claim 22, wherein the first determining module is configured to:
determine the target physiological parameter of the user based on at least one of the user scenario information and historical vital sign data of the user.

24. The apparatus of claim 22 or 23, wherein the first determining module is configured to:

in response to the user scenario information indicating the sleep aid scenario or the focus scenario, determine target activity of the user; or

in response to the user scenario information indicating the weight loss scenario, determine at least one of target activeness, target activity amount or target heart rate of the user; or

in response to the user scenario information indicating the emotion regulation scenario, determine heart rate variability of the user.

25. The apparatus of any one of claims 22 to 24, wherein, the target physiological parameter comprise activity, and the apparatus further comprises a second determining module configured to:

obtain historical heart rate data and historical activity data of the user within a third time range corresponding to the first time range;

determine the historical activeness of the user based on the historical heart rate data and the historical activity data of the user; and

determine target activeness of the user based on the historical activeness of the user, wherein the target activeness is less than the historical activeness.

26. The apparatus of any of claims 22 to 25, wherein the first determining module is configured to:
input the predicted vital sign data, the target physiological parameter and the user attribute information into a regression model, to obtain the one or more target audio parameters outputted by the regression model, wherein the one or more target audio parameters comprise target audio loudness and target rhythm.

27. The apparatus of any one of claims 18 to 26, wherein the acquiring module comprises:

a querying unit, configured to send a target audio modulation parameter to a server, so as to enable the server to query target audio data matching the target audio modulation parameter; and

a processing unit, configured to obtain the target audio data sent by the server.

28. A wearable device, comprising:

at least one processor; and

a memory communicatively coupled to the at least one processor; wherein

the memory is stored with instructions executable by the at least one processor, and execution of the instructions by the at least one processor causes the at least one processor to perform the method of any of claims 1 to 10 or the method of any of claims 11 to 17.

29. A computer readable storage medium having stored therein computer executable instructions that, when executed by a processor, causes the processor to perform the method of any one of claims 1 to 10 or the method of any of claims 11 to 17.

30. A computer program product comprising a computer program, wherein when the computer program is executed by a processor, the method of any of claims 1 to 10 or the method of any of claims 11 to 17 is performed.

obtaining measured vital sign data of a user within a first time range — S101

performing a vital sign prediction based on the measured vital sign data within the first time range, to obtain predicted vital sign data of the user within a second time range after the first time range — S102

determining one or more target audio parameters based on the predicted vital sign data and user attribute information of the user — S103

obtaining matched target audio data based on the one or more target audio parameters — S104

FIG. 1

user vital sign data
(heart rate, activity
amount, pressure, sleep,
emotion and respiratory
rate)

determining one or more target audio parameters based
on measured vital sign data and user attribute
information of a wearable device

measured vital sign
data
(heart rate, activity
amount, pressure,
sleep, emotion and
respiratory rate)

set vital sign data
（sleep aid scenario：
activeness
loss-weight-by-exercise
scenario：fat burning heart rate
emotion regulation scenario：
HRV）

user attribute
information
（name, age, height,
weight）
（weather、time）

determining one or more target audio parameters

audio
demand
matching

generating audio modulation library based on audio modulation rules

audio modulation rules
(rhythm and loudness)

original audio elements
(drum points, melodies,
natural sounds and volume)

modulating audio to store
in a libary

audio
modulation
library in
clound

drum point set (M)
modulated drum points 1...
modulated drum points M

melody set (M)
modulated melody 1...
modulated melody M

natural sound set (M)
modulated natural sound 1...
modulated natural sound M

volume set (M)
modulated volume 1...
modulated volume M

audio playing

matching audio demand and playing audio

FIG. 2

in response to the user scenario information indicating the sleep aid scenario, determining a target physiological parameter corresponding to the sleep aid scenario based on historical vital sign data of a user — S301

collecting measured vital sign data of the user within a first time range — S302

inputting the measured vital sign data within the first time range into a shared layer of a prediction model for feature extraction, to obtain the first feature data — S303

fusing the first feature data and user attribute information to obtain second feature data — S304

inputting the second feature data into the prediction layer of the prediction model for prediction, to obtain the predicted vital sign data within the second time range — S305

inputting the predicted vital sign data, the target physiological parameter and the user attribute information into a regression model, to obtain one or more target audio parameters outputted by the regression model — S306

querying matched target audio elements in the server based on the one or more target audio parameters — S307

synthesizing the queried target audio elements to obtain the matched target audio data — S308

FIG. 3

shared layer of prediction model | prediction layer of prediction model

```
monitored                                        independent
data of heart  ──▶  feature                      prediction          ──▶  prediction
rate                extraction                    layer                   data of heart
                    layer of                      corresponding            rate
monitored           neural        fusion          to heart rate
data of       ──▶   network
activity                                          independent
amount                                            prediction          ──▶  prediction
                    user                           layer                   data of
                    attribute     ──▶              corresponding            activity
                    information                    to activity              amount
                                                   amount
```

FIG. 4

```
measured vital sign data
(heart rate, activity
amount, pressure, sleep,
emotion and respiratory
rate)

set vital sign data                regression model                  model output
(sleep aid scenario:         ──▶   machine learning: XGBoost,   ──▶  target audio loudness,
activeness                         SVR, NN                            the target rhythm
weight-loss-by-exercise            deep learning: multi-layer
scenario: fat burning              residual network
heart rate
emotion regulation
scenario: HRV)

user attribute information
(name, age, height,
weight)
(weather, time)
```

FIG. 5

obtaining a plurality of historical data samples ⌐ S601

for each history data sample, labeling the target audio parameters in the each history data sample to obtain labeled target audio parameters ⌐ S602

obtaining the predicted audio parameters outputted by the regression model ⌐ S603

training the regression model based on a difference between the predicted audio parameters and the labeled target audio parameters, so as to minimize the difference ⌐ S604

collecting measured vital sign data of the user within a first time range ⌐ S605

performing a vital sign prediction based on the measured vital sign data within the first time range, to obtain predicted vital sign data of the user within a second time range after the first time range ⌐ S606

inputing the predicted vital sign data, the target physiological parameter and the user attribute information into the regression model, to obtain the one or more target audio parameters outputted by the regression model ⌐ S607

obtaining matched target audio data based on the one or more target audio parameters ⌐ S608

FIG. 6

71

collecting module

72

predicting module

73

first determining
module

74

acquiring module

FIG. 7

75

second determining module

71

collecting module

72

prediction module

721

extracting
unit

722

fusing unit

723

predicting
unit

73

first determination module

74

acquiring module

741

querying
unit

742

processing
unit

FIG. 8

FIG. 9

## INTERNATIONAL SEARCH REPORT

| International application No. |
|---|
| **PCT/CN2022/114355** |

**A. CLASSIFICATION OF SUBJECT MATTER**

A61M 21/00(2006.01)i; A61M 21/02(2006.01)i; A61B 5/0205(2006.01)i; A61B 5/02(2006.01)i; G06F 17/40(2006.01)i; G06F 17/00(2019.01)i; G06F 16/60(2019.01)i

According to International Patent Classification (IPC) or to both national classification and IPC

**B. FIELDS SEARCHED**

Minimum documentation searched (classification system followed by classification symbols)

A61M; A61B; G06F

Documentation searched other than minimum documentation to the extent that such documents are included in the fields searched

Electronic data base consulted during the international search (name of data base and, where practicable, search terms used)

CNABS; CNTXT; CNKI; VEN; USTXT; EPTXT; WOTXT: 华米健康, 黄汪, 汪孔桥, 严纪年, 朱国康, 孟孜, 张聪, 俞轶, 音频, 音乐, 歌曲, 声音, 乐曲, 体征, 生理参数, 生理信号, 预测, 预估, 推断, 推定, 推测, 心率, 脉搏, 压力, 睡眠, 体温, 情绪, 呼吸, 活动, 运动, 减肥, 减重, 锻炼, 专注, 工作, 助眠, 睡觉, 入睡, 入眠, 位置, GPS, biometric+, biolog+, sound, music, audio, predict+, heart rate, mood, emotion+

**C. DOCUMENTS CONSIDERED TO BE RELEVANT**

| Category* | Citation of document, with indication, where appropriate, of the relevant passages | Relevant to claim No. |
|---|---|---|
| X | CN 107272900 A (叶富阳 (YE, Fuyang)) 20 October 2017 (2017-10-20) description, paragraphs [0002]-[0018], and figures 1 and 2 | 11, 12, 17, 28-30 |
| Y | CN 107272900 A (叶富阳 (YE, Fuyang)) 20 October 2017 (2017-10-20) description, paragraphs [0002]-[0018], and figures 1 and 2 | 1-10, 13-30 |
| Y | US 10387173 B1 (INTUIT INC.) 20 August 2019 (2019-08-20) claims 1-30, and description, column 3, line 34-column 8, line 49 | 1-10, 13-30 |
| X | US 2018088895 A1 (SONOS INC.) 29 March 2018 (2018-03-29) description, paragraphs [0015]-[0024] and [0085]-[0163] | 11, 12, 17, 28-30 |
| Y | US 2018088895 A1 (SONOS INC.) 29 March 2018 (2018-03-29) description, paragraphs [0015]-[0024] and [0085]-[0163] | 1-10, 13-30 |
| Y | CN 111247782 A (SAMSUNG ELECTRONICS CO., LTD.) 05 June 2020 (2020-06-05) claims 1-6, description, paragraphs [0041]-[0043] and [0087]-[0092], and figures 1-17 | 1-10, 13-30 |
| A | US 2010186577 A1 (SAMSUNG ELECTRONICS CO., LTD.) 29 July 2010 (2010-07-29) entire document | 1-30 |

☑ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **24 October 2022** | **16 November 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/114355**

**C.    DOCUMENTS CONSIDERED TO BE RELEVANT**

| Category* | Citation of document, with indication, where appropriate, of the relevant passages | Relevant to claim No. |
|---|---|---|
| A | US 2006167576 A1 (OUTLAND RESEARCH, L.L.C.) 27 July 2006 (2006-07-27)<br>    entire document | 1-30 |
| A | US 2016339300 A1 (EBAY INC.) 24 November 2016 (2016-11-24)<br>    entire document | 1-30 |
| A | CN 106599057 A (SHANGHAI PHICOMM COMMUNICATION CO., LTD.) 26 April 2017<br>(2017-04-26)<br>    entire document | 1-30 |
| A | CN 105930455 A (CHENGDU TUYAN TECHNOLOGY CO., LTD.) 07 September 2016<br>(2016-09-07)<br>    entire document | 1-30 |

Form PCT/ISA/210 (second sheet) (January 2015)

**INTERNATIONAL SEARCH REPORT**
Information on patent family members

| International application No. |
| --- |
| **PCT/CN2022/114355** |

| Patent document cited in search report | | | Publication date (day/month/year) | Patent family member(s) | | | Publication date (day/month/year) |
| --- | --- | --- | --- | --- | --- | --- | --- |
| CN | 107272900 | A | 20 October 2017 | None | | | |
| US | 10387173 | B1 | 20 August 2019 | None | | | |
| US | 2018088895 | A1 | 29 March 2018 | US | 10318233 | B2 | 11 June 2019 |
| CN | 111247782 | A | 05 June 2020 | WO | 2019088734 | A1 | 09 May 2019 |
| | | | | US | 2019130364 | A1 | 02 May 2019 |
| | | | | EP | 3669536 | A1 | 24 June 2020 |
| | | | | EP | 3669536 | A4 | 02 September 2020 |
| | | | | US | 11321673 | B2 | 03 May 2022 |
| US | 2010186577 | A1 | 29 July 2010 | KR | 20100086618 | A | 02 August 2010 |
| | | | | KR | 101142679 | B1 | 03 May 2012 |
| US | 2006167576 | A1 | 27 July 2006 | US | 7542816 | B2 | 02 June 2009 |
| | | | | GB | 2437221 | A | 17 October 2007 |
| US | 2016339300 | A1 | 24 November 2016 | None | | | |
| CN | 106599057 | A | 26 April 2017 | None | | | |
| CN | 105930455 | A | 07 September 2016 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210008594